Europäisches Patentamt

**European Patent Office**

**Office européen des brevets**

(19)

(11) Publication number: **0 113 075**
**A2**

(12) **EUROPEAN PATENT APPLICATION**

(21) Application number: **83112249.4**

(22) Date of filing: **06.12.83**

(51) Int. Cl.³: **G 01 N 33/54**
**C 12 Q 1/28**

(30) Priority: **06.12.82 AU 7134/82**

(43) Date of publication of application:
**11.07.84 Bulletin 84/28**

(84) Designated Contracting States:
**AT BE CH DE FR GB IT LI NL SE**

(71) Applicant: **FIELDER GILLESPIE DAVIS LIMITED**
**55 Clarence Steet**
**Sydney New South Wales(AU)**

(72) Inventor: **Bundesen, Peter Gregory**
**15 Laura Street**
**Ekibin Queensland 4121(AU)**

(72) Inventor: **Rylatt, Dennis Brian**
**77 Elizabeth Street**
**Rosalie Queensland 4067(AU)**

(72) Inventor: **Wyatt, David Michael**
**39 Suvla Street**
**Balmoral Queensland 4171(AU)**

(72) Inventor: **Welch, John Sinclair**
**34 D'Aguilar Road**
**The Gap Queensland 4061(AU)**

(74) Representative: **Brown, John David et al,**
**FORRESTER & BOEHMERT Widenmayerstrasse 4/1**
**D-8000 München 22(DE)**

(54) **Field immunoassay reaction system, method of immunoassay diagnosis and probe or carrier for use therewith.**

(57) A field immunoassay reaction system and method is provided. The reaction system comprises the following components:

   (i)  a dipstick carrier or probe at least partly coated with monoclonal antibody specific for an antigen contained in a body substance or fluid;

   (ii)  a plurality of containers;

   (iii)  detergent or other suitable substance to prevent non-specific binding of antigen to the monoclonal antibody;

   (iv)  buffer; and

   (v)  in situ signal amplification or detection means.

   Preferably there is also included as an additional component a second monoclonal antibody labelled with an enzyme and enzyme substrate. The detection means is suitably a colour standard or portable colourimeter.

COMPLETE DOCUMENT

Croydon Printing Company Ltd.

FIG.1

Title:  Field immunoassay reaction system, method of
         immunoassay diagnosis and probe or carrier for
         use therewith.

THIS INVENTION relates to a field immunoassay for use as diagnosis of antigens in body substances.

Immunoassays have revolutionized human diagnostic medicine and veterinary medicine since the introduction of sensitive quantitative techniques such as the radioimmunoassay (RIA) first reported by Yallow and Berson Nature 184 1648 1959 and the enzyme immunoassay or EIA also including the ELISA technique which were first reported in Engvall and Perlmann Immunochem 8 871 (1971) and Van Weeman and Schuurs FEBS Letters 15 232 (1971). Both these assay systems depend on the exquisite specificity of the antigen/antibody reaction. After the advent of the competitive RIA in 1959, a number of different types of RIA have been developed and because the underlying principles of the heterogenous EIA are the same as those for RIA, EIA's analogous to existing RIA's have been devised and these would include competitive assays, immunometric assays, two site immunometric assays, sandwich assay and double Ab immunometric assays.

Both EIA and RIA can be designed to detect either antibody (Ab) or antigen (Ag) in a suitable test solution. The following invention addresses and relates to only one of these aspects, that of Ag detection in a suitable solution.

In general terms, the detection of an Ag in a suitable fluid by an immunoassay system depends on the specific interaction between Ab and Ag, to form the Ab/Ag complex which must be separated from free Ab and Ag to allow the detection and quantitation of the Ab/Ag complex. The detection of these complexes can be achieved by various immunoassay strategies but most involve as a prior step a suitable label being coupled to either (a) Ag or (b) Ab.

In regard to immunoassays employing labelled Ag, these can be performed either in solution or with an appropriate solid phase but all these assays share one common problem - the production of highly purified 'tracer grade' antigen. Preparation of such an antigen is often expensive

2.

and difficult and in some cases the combination of both of these factors may render the development of a labelled Ag based assay virtually impossible for commercial application.

In relation to immunoassays employing labelled Ab, this type of assay is generally performed on or with some suitable form of solid phase coupled Ag or Ab. However, out of all the possible combinations of assay systems available only the 'Two Site Assay' eliminates the necessity to obtain a purified Ag with all its attendant problems.

The 'Two Site Assay' can only be performed on an Ag able to bind simultaneously two Ab molecules. In this assay specific Ab is coupled to a suitable solid phase and a solution containing the Ag is incubated with the sensitized surface allowing Ag to be 'captured'. The excess fluid is washed away and a conjugate consisting of a second labelled Ag specific Ab is added and this labelled Ab has the capacity to 'tag' the captured Ag on the solid phase.

The advantages of the two site assay are manifold:

(i)     they do not require purified antigen,

(ii)    they can be employed with any substance that is, or can be made, immunogenic to an experimental animal,

(iii)   they can be employed with any immunogenic substance that can be presented in an aqueous solution,

(iv)    with suitable Ab, the Ag solution and the 'tagging' Ab can be added simultaneously to the solid phase 'capture' Ab, thus providing an extremely rapid and simple assay system.

A serious limitation of the 'Two Site Assay' system is the preparation of sufficient quantities of suitable 'capture' and 'tag' Ab (polyclonal antibody) and this is particularly relevant with certain Ag's or Ag determinants that are poor immunogens. This is of great importance in the production of commercial assays and has only recently been successfully addressed by employing hybridoma technology, allowing the enormous potential of the 'Two Site Assay' to be

3,

fully exploited. Hybridoma technology has allowed the production of virtually unlimited quantities of 'capture' and 'tag' monoclonal antibodies (MAb) of unparalleled antigen specificity and antigenic determinant specificity. The key provided by hybridoma technology resides in the production of MAb that can be employed specifically as the 'capture' MAb, which bind a unique antigenic determinant only present on the test Ag and absent on any other molecular species. Thus MAb 'capture', 'tag' assays can now be constructed that were previously impossible due to the lack of Ag determinant specificity with conventionally produced polyclonal antisera (PAb), which resulted in cross-reactions with molecules having similar antigenic determinants.

Various types of solid phase surfaces have been employed in relation to two site immunoassays and these include test tubes having an open top wherein antibody was coated to the bottom surface of the test tube. However, problems with the use of test tubes in this manner were that this resulted in using excess amounts of capture Ab because of the large surface area to be coated. Difficulties were also encountered in washing and flushing out non bound material from the coated portion of the test tube. Related to this is the detection of signal amplification step where the background signal may be excessively high providing problems in efficient and correct interpretation of results.

Plastic beads or Sepharose beads were also utilized but a major problem with such beads was that a centrifugation step was required to remove unbound material and this was time consuming and inefficient in relation to large sample numbers.

Recently microtitre plates employing a plate with 48 or 96 wells have been utilized but while such plates have been efficient in relation to large numbers of samples being tested they were inefficient in regard to single tests or small numbers of tests especially in adverse conditions as would be experienced in field conditions.

Also other problems with conventional methods 0113075 immunoassay which have been mainly based on EIA such as ELISA as well as RIA as described above, fluroimmunoassay (FIA) or chemiluminescence include the necessity for electrical or electronic signal amplification systems which were expensive or alternatively not of convenient access in relation to relatively small laboratories or testing stations.

There is a clearly recognisable need for the development of an assay system that allows single use under field conditions and eliminates the problems of washing (cellulose acetate discs; plastic beads) or the excess use of the biological reagents (tube) and combines monoclonal antibody technology to allow the full potential of the Two Site Assay to be realized.

It has also often been the desire of medical practitioners, veterinarians, students, laboratory technologists and the like as well as semi-skilled people such as farmers, farmhands or nurses to have a quick and effective immunoassay method or diagnostic method available which could be utilized from an available test kit comprising diagnostic reagents whereby a fluid sample from animals as well as humans may be tested for signs of a particular disease without the necessity for transporting the fluid sample to a laboratory. Thus so far as method of immunoassay such as an EIA for use in an "in the field" or "off the cuff" situation was not available.

U.S. Patent 4 294 817 relates to an immunoassay method using two test surfaces mounted on single sampler which required analysis in a particular type of fluorometer. The sampler was passed sequentially through a test serum sample, washing solution, a solution containing immunofluorescent antibody and lastly through a final washing solution. The fluorescent measurements of the two surfaces were then subtracted to give a difference measure which was compared with difference meansurements obtained from control serum solutions. However, not only was a particular type of fluormeter normally required to give the abovementioned difference measurements, but a microcomputer accessory was usually also required. Thus although this test was relatively effective it did require the use of relatively sophisticated signal amplification apparatus.

Also the sampler used in the abovementioned immuno-assay comprised circular discs of a special porous material which was a copolymer of cellulose nitrate and cellulose acetate which were attached or mounted to a rod or tube by adhesive tape or suitable bonding agent. Such a process was extremely time consuming in requiring that after application of the antigen or antibody to the discs that they had to be dried for a relatively long time under specific conditions. Also the discs were only suitable for certain specific antibodies or antigens as described in U.S. Patent 4 294 817. The cellulose discs also bound a lot of non-specific material because of their porous nature and thus providing problems in obtaining an efficient washing technique.

Recently Australian Patent Specification 68117/81 was published describing a method of immunoassay which included the steps of:

  (i)  contacting a sample containing an unknown antigen or antibody with the internal surface of a cap having attached thereto antibody corresponding to the antigen in the sample;

  (ii)  contacting the cap with an appropriate enzyme conjugate; and

6.

(iii) contacting the cap with the enzyme substrate.

The internal surface of the cap could have projecting portions extending outwardly therefrom or separate elements attached to or retained within the cap, all of which may have the relevant antigen or antibody attached thereto.

The abovedescribed technique is stated as being useful for performance under adverse conditions or in the field such as diagnosis of snake bite. However, under practice it was found that it was necessary when attaching a cap having the antigen or antibody attached to the internal surface thereof to a container containing an unknown sample that it was necessary to invert the container so that it was standing on its cap, so that an appropriate reaction could take place so that the antigen or antibody in the sample could bind to the relevant antibody or antigen attached to the internal surface of the cap. It is believed that this procedure is disadvantageous in that difficulty will be experienced in achieving proper washing of the cap after the reaction. Thus use of a threaded cap as described in Patent 68117/81 will provide difficulties in washing or flushing out the reagents from the internal surface of the cap.

Thus it must be borne in mind that any immunoassay method in order to be reliable must demonstrate an efficient washing technique. If the washing procedure between subsequent steps of an immunoassay are not efficient errors could be introduced into the final results thereby providing an incorrect result.

In Patent Specification 68117/81 contact will occur between mating surfaces of both the cap and the container containing reagent or sample and thus liquid could travel to a location between the mating surfaces of cap and container by direct leakage if the cap was not firmly attached to the container. Alternatively such leaking could occur on the basis of capillary action or in some cases by surface tension or pressure gradients. This disadvantage will be most marked when use is made of a screw threaded cap as described in Patent specification 68117/81 followed by the step of inversion of the container so it stands on its cap to achieve a reaction between antibody and antigen. Washing steps are also accompanied by shaking of the container and to prevent leakage of liquid the cap must be very firmly sealed to the container.

Thus because of these factors the reproducibility of an assay carried out in accordance with Specification 68117/81 must be questionable because of the danger of contaminants such as impurities or foreign antigens interfering with the antigen-antibody reaction where such contaminants may be present because of an imperfect washing step. The susceptibility of contamination will be compounded as a result of the two washing steps that are described in relation to Patent Specification 68117/81.

It also must be borne in mind that when inexperienced personnel are carrying out the immunoassay method that contamination as described above is much more likely to occur.

In relation to use of a cap having a depending portion such as a tube or rod as described in Patent 68117/81 the only specific example given is that of a "dropper-type" cap having a rubber teat and depending tube which requires a pressure differential or pumping action to draw liquids up into the tube or be expelled therefrom. The difficulty of washing such a cap will be increased upon comparison with a normal screw threaded cap and thus the abovementioned disadvantages will be compounded. The cost of using such a cap will also be relatively high as will be the case of producing a one piece plastics moulding of cap and depending rod or tube. It also will be appreciated that if the depending rod or tube is separate from the cap that it will have to be bonded thereto with a suitable adhesive and there is a danger of contamination occuring if liquid gains access to the area between the mating surfaces of cap and depending rod or tube.

8.

It will also be appreciated that the cap with depending rod or tube as described in Patent Specification 68117/81 can only be used in relation to containers having a relatively large access mouth and thus such a cap would not be appropriate with containers having a narrow or restricted access mouth which would render adequate washing procedures very difficult due to enhanced capillary and surface tension effects.

Another disadvantage of the process described in Patent Specification 68117/81 is that it is adapted for single applications and thus is not easily adaptable for assay of a multiplicity of antigens in the same manner as the present invention as described hereinafter.

Also the process of Patent Specification 68117/81 has the necessity for provision of a water tight seal between cap and container which is not necessary in the present invention which is of a more simplified nature.

Recently as described in Czech. papers 2bl. Bakt Byg.I.Abt. Orig A 240 112-117 (1978) and 2bl. Bakt. Byg. I. Abt. Orig A 240 118-122 (1978) there have been described a case wherein a polystyrene stirrer having a rounded end is used as an antigen carrier wherein initially the rounded end of the stirrer is immersed in an antigen solution. For mass coating the stirrers or "sticks" are bundled in blocks and stuck through meshes formed by rubber bands which are arranged crosswise in two levels about 2 cm apart. After the bundles of sticks have been placed overnight in an antigen trough and after drying they are washed and then placed in tubes having falling Ag concentrations. The Ag coated sticks are then placed in a block of tubes containing serum dilutions and subsequently incubated. The sticks after washing are then placed in a block of tubes containing enzyme conjugated anti-human immunoglobulin. Following incubation the sticks are then washed and transferred to tubes filled

9.

with enzyme substrate solution. The enzyme reaction is then stopped by removal of the sticks from the substrate. The
substrate tubes then have readings taken photometrically.

However the Czech papers only describe the use of
stock carriers as applied to carrying antigens and not antibodies or MAb and a multiple use is only envisaged.

In regard to Australian Patent 521002 this describes
the use of a stick or insert type assay using an insert matrix
of a variety of complicated shapes comprising a plurality of
fins such as those shown in the drawings. It is also noted
that all the finned matrices are adapted to conform closely
to the internal surface of the sample container. Not only would
the finned inserts be difficult and expensive to manufacture
but it would seem that such inserts would be unsuitable for
field or barefoot assays because of the difficulty in washing
the inserts due to poor drainage due to capillary adherence
of the reaction fluid. This problem is already specifically
referred to in this Patent.

Patent 521002 is clearly only directed to laboratory
type assays due to the character of the matrix inserts as
described and does not have any reference to the use of monoclonal antibodies as in the two-site assays described previously.
Reference is only made to polyclonal species. There is also
no reference to the applicability of the assay procedure to
barefoot assays or to reaction kits for use therein.

In regard to field EIA immunoassays or "barefoot"
immunoassays such assays have not been very well developed
and have had failings insofar as lack of specificity by use
of polyclonal species and also by the necessity for results
to be considered significant must be carried out under
laboratory conditions. The abovementioned Czech papers
only described mass coating of sticks and even then the
coatings were derived from antigens or polyclonal species.

Therefore an object of the invention is to provide a
method and reaction system useful in field immunoassays which

10.

enables such immunoassays to be efficient in use and provide accurate results especially where access to a laboratory is not available.

The present invention is based on the discovery that field immunoassays may be carried out efficiently and accurately by coating an elongate probe with monoclonal antibody specific for an antigen contained in a body substance or fluid.

Therefore in one aspect the present invention provides a field immunoassay reaction system for "in situ" testing comprising:

(i)     a dipstick probe or carrier at least partly coated with monoclonal antibody (MAb) specific for an antigen contained in a body substance or fluid;

(ii)    a plurality of containers;

(iii)   detergent or other substance to prevent non-specific binding of antigen to monoclonal antibody;

(iv)    buffer;  and

(v)     "in situ" signal amplification or detection means.

The invention also provides a dipstick probe or carrier at least partly coated with monoclonal antibody (Mab) specific for an antigen contained in a body substance or fluid, suitable for use in the aforementioned field immunoassay reaction system for "in situ" testing.

Suitable materials from which the dipstick probe or carrier may be formed comprise substantially non-porous materials which are absorbent insofar as the MAb are concerned. Suitably such materials are non-porous. Of these materials plastics are preferred and suitable examples are polystyrene, acrylics and modified acrylics as well as polyamides and nylon.

The dipstick member may comprise a rod or a tube which is open at each end. The MAb is applied to the insertable part which of course is accessible to antigen in a sample container and this may comprise the external surface of the rod or the interior and exterior surface of the open ended tube. However use of a rod is preferred.

It is also preferred that an insertable part of the dipstick carrier may be enlarged in relation to a handle part and be provided with one or more ribs, grooves, outwardly extending projections or cavities or combinations thereof to substantially increase the surface area thereof so as to enhance the coating of MAb thereon.

The tube or rod may be any suitable shape and cross-section and thus be round or rectangular in cross-section. Preferably, however, the tube or rod is multi-faceted and thus be hexagonal or polygonal in cross-section.

Prior to assembling the reaction system or kit the insertable part of the dipstick carrier may be immersed in a solution of MAb for an appropriate length of time (eg. 15 minutes or greater) to allow attachment of the MAb thereto. The MAb may be passively or actively coated onto said insertable part of the dipstick member. The MAb may be attached to the dipstick member on the basis of hydrogen bonding, Van der Waals forces, covalent bonding in some cases or other mutually attractive forces between the elongate member and MAb. Suitably this step is carried out at room temperature although a normal incubation temperature and appropriate physiological conditions (eg 27-37°C) may be attained. If desired the MAb may be dried on the dipstick member surface such as by

11.

freeze drying.

Subsequently the dipstick carrier may be inserted into a container containing the sample body substance and an antigen contained in the sample body substance may then be bound to the coated capture MAb. After withdrawal the dipstick member is washed with an appropriate washing agent such as phosphate-buffered saline (PBS). The washing step may be repeated. The detection of the captured antigen may be carried out using a second enzyme conjugated tag MAb which couples to the capture MAb-antigen reaction product. After addition of a substrate as one example of signal amplification technique the amount of unknown antigen in the sample may be determined by an EIA technique.

The contact of the coated dipstick carrier with the sample substance may be carried out at room temperature. Appropriate washing agents are PBS (phosphate-buffered saline) or a mixture of PBS and Tween. Suitably the abovementioned contacting step is carried out between a pH of 5 to 9. A suitable upper limit of ionic strength is 1M. These conditions also may apply to the bonding of MAb to the elongate carrier.

Using the process of the invention it normally should not be necessary to use any concentrating steps to concentrate the sample for assay. Thus a larger volume of sample may initially be obtained to overcome any initial concentration of sample.

A wide variety of antigens may be analysed by the process of the invention and these include antigens contained in animal body or fluid substances (including human body substances or fluids such as faeces, lymph, urine, blood including components thereof such as blood plasma and blood serum, blood coagulum or fibrin, saliva, extracellular fluid, cereliospinal fluid, tissue homogenates or solution prepared from any of the foregoing). In particular the invention is directed at the diagnosis of animal body excretions and secretions.

Preferably the labelling enzyme used for the second MAb is selected from the group comprising a peroxidase, beta galactosidase, alkaline phosphatase and urease. Suitable enzyme substrates for a peroxidase include O-phenyldiamine, O-tolidine or ABTS (ie 2-2 azino-di-(3 ethyl benzothiazolin sulphone 6). Suitable enzyme substrates for beta galactosidase include para or ortho nitrophenol-β-D-galactosidase. In relation to urease a suitable substrate is urea and in relation to alkaline phosphatase a suitable substrate is paranitrophenol phosphate.

Suitably in the reaction system of the invention the second MAb labelled with the enzyme is freeze dried or is otherwise prepared for use in the field.

In relation to the use of peroxidases hydrogen peroxide must also usually be present as this is a necessary cofactor.

As an alternative instead of labelled second MAb, if the antigen captured by the MAb on the elongate carrier is chemically active, the signal amplification step may be achieved directly by allowing the captured antigen to initiate a detectable chemical reaction with the appropriate reagent or reagents.

Preferably the buffer component which may be used in the present invention is PBS although any other suitable buffer may be used to suitably provide a pH of approximately 7 such as citrate buffer or lactate. The buffer may be supplied in the form of a powder in a kit although it may be supplied as a liquid if desired.

Preferably the detergent component is polyoxyethylene sorbitan monolaurate although other suitable detergents will be apparent to the man skilled in the art. The detergent component is necessary to prevent non-specific binding to the MAb coated probe or carrier. The detergent may be supplied in dry form such as being impregnated onto filter paper although it may be supplied as a liquid.

If required a germicidal or bacteriocidal component may be used such as sodium azide or ethyl mercurithio salicylate. If the buffer is to be supplied in liquid form it should be combined with the bacteriocidal agent. The bacteriocidal component however may be omitted if the reaction system of the invention is packaged in a sterile manner.

Preferably the containers utilized are test tubes and these are colour coded or provided with other suitable identifying means to distinguish one tube from the remainder. Some of the test tubes will only be used for washing purposes as the elongate carrier is transferred from one container to another and some of the test tubes may be used for containing certain reactants such as the sample or enzyme labelled second monoclonal antibody.

Suitably the components such as the enzyme substrate are provided in separate vials or ampoules.

Preferably when using an EIA test as a signal amplification step or detection step a colour standard or portable colourimeter may also be utilized to check the results of the test.

Preferably also one or more loop dilutors are provided so that transfer of sample or reagent to a test tube containing dilutent may be effected.

Reference is now made to detailed experimental procedures illustrating the immunoassay technique of the invention. In the examples referred to hereinafter the undermentioned reagents for use in the reaction system or kit were prepared as follows:

Phosphate-buffered Saline (PBS) powder

A mixture of o.16 g Sodium chloride, 0.004 g Potassium chloride, 0.023 g Disodium hydrogen phosphate and 0.004 g of Potassium dihydrogen phosphate.

Tween Impregnated Paper

this reagent was prepared by spotting 100 ul of 10% Tween 20 (polyoxyethylene sorbitan monolaurate) on to a strip of filter paper and allowing to dry in air.

Enzyme Substrate Solution

This was prepared from 50 m M citrate, 2.5 m M 0-tolidine dihydro chloride and 0.025 m M EDTA, pH 4.5.

Peroxidase-conjugated Monoclonal Antibody

This was prepared from the following procedure:

5 mg horseradish peroxidase obtained from Calbiochem Laboratories was dissolved in a 1.0 ml distilled water and 0.2 ml of 0.1 M NaIO₄ added. A colour change occurred from gold to green. The mixture was mixed gently for 20 minutes at room temperature. Free NaIO₄ was removed by gel filtration. The gel filtration column was equilibrated with 0.001M Citrate buffer pH 4.5. Monoclonal antibody prepared from human D dimer or dog heartworm antigen as described hereinafter in Examples 1 and 2, was added in a ratio of 1 mg HRPO to 2 mg immunoglobulin. One M Na₂CO₃ buffer at pH 9.5 was added to give a final pH of between 9.0 - 9.5. This is an optimum pH for coupling of aldehydes to amine groups. The resulting mixture was mixed gently for 2 - 3 hours at room temperature. Then 200 ul of 2.0 M ethanolamine was added and the pH adjusted to 9.5. The mixture was then incubated overnight at 4°C. Ethanolamine was removed by gel filtration and Methiolate (0.01%) ethylmercuritho salicylate was added and the conjugated antibody stored at 4°C. For final kit preparation conjugated MAb was diluted 1/10 in 10% sucrose and 10 ul of this solution was freeze fried in the appropriate tube.

Probes were prepared by immersing polystyrene sticks having an enlarged plate-like body portion with a plurality of transverse ribs (e.g. 6-8) located on both broad surfaces thereof down to a depth of 2 cm in the relevant purified MAb (10µg of protein/ml) for one hour at room temperature. The coated stick was then washed three times by dipping to a depth of 3 cm in PBS/Tween, leaving for 5 minutes and then transferring to another container of PBS/Tween. The washed sticks were then immersed to a depth of 3 cm in a solution of bovine serum albumen to take up unreacted binding sites (Sigma A4503) 10mg/ml in PBS for a further 1 hour at room temperature and washed a further 3 times in PBS/Tween. Finally the sticks were flicked to remove excess liquid and allowed to air dry.

EXAMPLE 1

Detection of human cross-linked fibrin derivatives in plasma for the determination of Disseminated Intravascular Coagulation (DIC)

In this particular example, reaction probes were coated with specific monoclonal antibody able to capture specific antigen from a suitable solution, and the captured antigen immobilized on the reaction probe was then subsequently tagged using a suitable labelled second monoclonal antibody.

Monoclonal antibodies to human D dimer antigen were prepared in accordance with the technique established by Galfre et al, Nature 266, 550-2 (1977) as follows:

(i)     Immunization of BALB/c mice with human D dimer antigen.

(ii)    Fusing spenic cells from immunized mice obtained in step (i) with NS-1 mouse myeloma cells where polyethylene glycol is used as a cell fusing agent to produce the hybridoma cells.

(iii)   Propogating the hybridoma cells in tissue culture to produce anti-D dimer monoclonal antibody secreting clones.

(iv)    Injecting the cloned bybridoma cells obtained n step (iii) into BABL/c mice wherein tumours are produced which secrete the anti-D dimer monoclonal antibody.

(v)    Collection of the monoclonal antibody from mice injected in step (iv).

Using this technique, the following anti-D dimer monoclonal antibodies were prepared:

(a)    clone B 42.7.3B6/22

(b)    clone B 44.7.4D2/182.

Reaction probes coated with B42.7.3B6/22 monoclonal antibody were prepared (as described previously).

## Kit description

The test kit consists of 3 x 2 ml empty test tubes or containers, one 2 ml red capped tube containing freeze dried horseradish peroxidase conjugated MAb B 44.7.402/182, one capped 20 ml dropper bottle with separate nozzle containing detergent impregnated paper strip and PBS buffer powder, a polystyrene stick coated with MAb B 42.7.3B6/22, a 50 ul dilutor, and colour coded sealed glass ampoules containing:

1.    green ampoule:    0.75 ml of 3% hydrogen peroxide

2.    yellow ampoule:    1 ml of O-tolidine substrate solution

3.    pink ampoule:    1 ml blue colour standard.

The complete kit also contains a cardboard holder for stationing of the reagents and a set of instructions.

Before the test for D dimer can be carried out some kit preparation is required.

1.    20 ml of distilled water was added to fill the dropper bottle containing paper strip and buffer powder.  The nozzle is inserted and the contents mixed.

2.    10 drops of buffer was added to the first empty tube. 50 ul of the test sample was then transferred with the aid of the dilutor and the contents were mixed by agitation with the loop for 10 sec.

3.    20 drops of buffer were added to the red capped tube and contents mixed by inversion.

ASSAY PROCEDURE

The test was carried out by the following required steps.

1.  The MAb coated probe was removed from its satchet and placed in the tube containing the diluted test sample. Leave 45 minutes.

2., 3., and 4 Buffer was added to the neck of the 2nd empty test tube and the probe was transferred to this tube for washing. After rotation for 30 seconds the probe was allowed to stand for 30 seconds. The probe was then removed and the contents of this tube discarded. The tube was again filled with fresh buffer. Thorough washing is essential. The washing procedure was repeated 2 more times.

5.  Transfer probe to red capped tube and leave 45 minutes.

6., 7., and 8.,

Transfer probe to the third uncapped tube and proceed as for 2, 3 and 4.

9.  Transfer probe to the green ampoule and leave 30 sec.

10. Transfer probe to yellow ampoule, rotate probe 30 sec and leave 5 minutes. The probe was discarded after 10 minutes.

When this ampoule was held against a clean white background the presence of a blue colour greater than the colour standard (pink ampoule) was determined. If the test sample contained the specific antigen, a blue colour was obtained which ranged from a faint to a dark blue and the degree of colour was proportional to the amount of antigen present. If a quantitative result was required in a laboratory the contents of the green ampoule could be transferred to a cuvette and the absorbance determined in a spectrophotometer at $A_{620nm}$. The results of an assay on human plasma samples are shown in Table 1 reproduced herein below. For the purposes of preliminary evaluation the colour standard was set to give a $A_{620nm}$ value of 0.100.

0113075

| Sample (1) | Probe Assay | | (2) D dimer ng/ml | (3) Confirmed DIC |
|---|---|---|---|---|
| | Visual Determination | Quantitative Determination $A_{620}$ | | |
| 1 | ++ | 0.415 | 200 | Yes |
| 2 | ++ | 0.445 | 120 | Yes |
| 3 | +++ | 0.736 | 450 | Yes |
| 4 | +++ | 0.887 | 620 | Yes |
| 5 | +++ | 0.753 | 280 | Yes |
| 6 | + | 0.252 | 80 | Yes |
| 7 | - | .05 | - | No |
| 8 | - | 0.044 | - | No |
| 9 | - | 0.026 | - | No |
| 10 | - | 0.045 | - | No |

(1)    -   no blue colour

        +   weak blue colour

       ++   medium blue colour

      +++   strong blue colour

(2)  Performed according to Procedures described by:  Rylatt, D.B. et al, Thrombos Res., 31, 767-778, 1983 and Elms, MJ, et al, Thromb Homeost, 50, 591-594, 1983.

(3)  DIC patents diagnosed according to the scoring system of Whaun, J.M. and Oski, F.A., Can Med. Assoc. J., 107, 963-967.

19.

EXAMPLE 2

The detection of D.immitis antigen using antibody coated reaction probes

In this particular example, reaction probes were coated with specific anti D. immitis MAb able to capture specific antigen from a suitable solution, and the captured antigen immobilized on the reaction probe was then subsequently tagged using a suitably labelled second anti-D-immitis monoclonal antibody.

Monoclonal antibodies to dog heartworm (Dirofilaria immitis) antigens were prepared in accordance with the technique established by Galfre et al, Nature 266, 550-2 (1977) as follows:

(i)      Immunization of BALB/c mice with D.immitis antigens,

(ii)     Fusing splenic cells from immunized mice obtained in step (i) with NS-1 mouse myeloma cells where polyethylene glycol is used as a cell fusing agent to product the hybrodoma cells.

(iii)    Propagating the hybridoma cells in tissue culture to produce anti-D.immitis monoclonal antibody secreting clones.

(iv)     Injecting the cloned hybridoma cells obtained in step (iii) into BALB/c mice wherein tumours are produced which secrete the anti-D.immitis monoclonal antibody.

(v)      Collection of monoclonal antibody from mice injected in step (iv).

Using this technique, the following anti-D.immitis monoclonal antibodies were prepared:

(a)      clone B 49.8.1A1/53 (A)

(b)      clone B 48.8.4A4/45 (B)

(c)      clone B 48.8.4C2/121(C)

Reaction probes coated with the above monoclonal antibodies (a), (b) or (c) were prepared (as described previously).

Assay for Heartworm Antigen

The test kit consists of 3 x 2 ml empty test tubes or containers, one 2 ml red capped tube containing either freeze dried horseradish peroxidase conjugated MAb A, B or C prepared as described in example one; one capped 20 ml dropper bottle with separate nozzle containing detergent impregnated paper strip and PBS buffer powder, a polystyrene stick covered with either MAb A, B or C, a 10 μl loop dilutor, and colour coded sealed glass ampoules containing:

1.       green ampoule 0.75 ml of 3% hydrogen peroxide

2.       yellow ampoule 1 ml of o-tolidine substrate solution

3.       pink ampoule: 1 ml blue colour standard

The complete kit also contains a cardboard holder for stationing of the reagents and a set of instructions.

Before the test for heartworm antigen can be carried out some kit preparation is required.

1.       20 ml of distilled water was added to fill the dropper bottle containing paper strip and buffer powder. The nozzle is inserted and the contents mixed.

2.       10 drops of buffer was added to the first empty tube. 10 μl of the test sample (heartworm antigen 200 μg/ml) was then transferred with the aid of the dilutor and the contents were mixed by agitation with the loop for 10 sec.

3.       20 drops of buffer were added to the red capped tube and contents mixed by inversion.

Assay Procedure

The test was carried out by the following required steps.

1.       The MAb coated probe was removed from its satchet and placed in the tube containing the diluted test sample. Leave 15 minutes.

2,3,4    Buffer was added to the neck of the 2nd empty test tube and the probe was transferred to this tube for washing. After rotation for 30 seconds the probe was allowed to stand

-21-

for 30 seconds. The probe was then removed and the contents of this tube discarded. The tube was again filled with fresh buffer. Thorough washing is essential. The washing procedure was repeated 2 more times.

5. Transfer probe to red capped tube and leave 15 minutes.

6,7,8 Transfer probe to the third uncapped tube and procede as for 2,3,4.

9. Transfer probe to the green ampoule and leave 30 sec.

10. Transfer probe to yellow ampoule, rotate probe 30 sec. and leave 5 minutes. The probe was discarded after 10 minutes.

When this ampoule was held against a clean white background the presence of a blue colour greater than the colour standard (pink ampoule) was determined. If the test sample contained the specific antigen, a blue colour was obtained which ranged from a faint to a dark blue and the degree of colour was proportional to the amount of antigen present. If a quantitative result was required in a laboratory the contents of the green ampoule could be transferred to a cuvette and the absorbance determined in a spectrophotometer at $A_{620nm}$. The results of an assay on human plasma samples are shown in Table 1 reproduced hereinbelow. For the purposes of preliminary evaluation the colour standard was set to give a $A_{620nm}$ value of 0.100.

TABLE 2

Results for Example 2

| Sample | Caputre Antibody | D.Immitis Antigen Present | Tag Antibody | EIA Value $A_{620nm}$ |
|--------|------------------|---------------------------|--------------|----------------------|
| U | Monoclonal Ab(a) | Yes | Monoclonal Ab(a) | 0.547 |
| V | Monoclonal Ab(b) | Yes | Monoclonal Ab(c) | 0.217 |
| W | Monoclonal Ab(c) | Yes | Monoclonal Ab(b) | 0.227 |
| X | Monoclonal Ab(a) | No | Monoclonal Ab(a) | 0 |
| Y | Monoclonal Ab(b) | No | Monoclonal Ab(c) | 0 |
| Z | Monoclonal Ab(c) | No | Monoclonal Ab(b) | 0 |

0113075

However, as previously stated, absorbance readings are not required and the assay can be carried out manually by inexperienced personnel by following the appropriate instructions.

From Example 2 above while the heartworm antigen was derived from adult heartworm tissue, it will be appreciated to those skilled in the art that the procedures described above in Examples 1 and 2 may be utilized for the detection and/or quantitation of circularing beartworm antigen contained in dog blood serum or plasma under certain conditions.

EXAMPLE 3

The detection of human immuno reactive trypsin using antibody coated reaction probes

In this example reaction probes were coated with human trypsin specific monoclonal antibodies able to capture human trypsin from a suitable solution and the captured antigen immobilized on the reaction probe was then subsequently tagged using a suitably labelled second anti-human trypsin monoclonal antibody.

Monoclonal antibodies to human trypsin were prepared in accordance with the technique established by Galfre et al, Nature 266, 550-2, 1977 as follows:

(i)     Immunization of BALB/c mice with human trypsin Calbiochem Cat. No. 650275.

(ii)    Fusing splenic cells from immunized mice obtained in Step (i) with NS-1 mouse myeloma cells wher polyethylene glycol is used as a cell fusing agent to produce the hybridoma cells.

(iii)   Propagating the hybridoma cells in tissue culture to produce anti-human trypsin monoclonal antibody secreting clones.

(iv)    Injecting the cloned hybridoma cells obtained in Step (iii) into BALB/c mice wherein tumours are produced which secrete the anti-human trypsin monoclonal antibody.

-23-

(v)     Collecting of monoclonal antibody from mice injected in Step (iv).

Using this technique the following anti-human trypsin monoclonal antibodies were prepared:

D  Clone C.13.13 2D1/182

E         C.11.13 3B6/140-157

F         C.20.13 3D6/184

Reaction probes were coated with the above monoclonal antibodies as described previously.  The assay kit as described in example 1 except that the red capped tube contained either horseradhish peroxidase conjugated MAb D or F.

Before the test for human immuno reactive trypsin can be carried out some kit preparation is required.

1.      20 ml of distilled water was added to fill the dropper bottle containing paper strip and buffer probided.  The nozzle is inserted and the contents mixed.

2.      10 drops of buffer were added to the first empty tube. Then a single blood spot 3 mm in diameter obtained as part of the neonatal screening program was added and the contents were allowed to elute for 2h at room temperature.

3.      20 drops of buffer were added to the red capped tube and contents mixed by inversion.

Assay Procedure

The rest was carried out by the following required steps.

1.      The MAb coated probe was removed from its satchet and placed in the tube containing the diluted test sample.  Leave 45 minutes.

2,3,4   Buffer was added to the neck of the 2nd empty test tube and the probe was transferred to this tube for washing. After rotation for 30 seconds the probe was allowed to stand for 30 seconds.  The probe was then removed and the contents of this tube discarded.  The tube was again filled with fresh buffer.  Thorough washing is essential.  The washing procedure was repeated 2 more times.

-24-

5.        Transfer probe to red capped tube and leave 45 minutes.

6,7,8    Transfer probe to the third uncapped tube and proceed
as for 2,3,4.

9.        Transfer probe to the green ampoule and leave 30 sec.

10.       Transfer probe to yellow ampoule, rotate probe 30 sec.
and leave 5 minutes.

The probe was discarded after 10 minutes.

When this ampoule was held against a clean white
background the presence of a blue colour greater than the
colour standard (pink ampoule) was determined. If the test
sample contained the specific antigen (trypsin), a blue
colour was obtained which ranged from a faint to a dark blue
and the degree of colour was proportional to the amount of
antigen present. If a quantitative result was required in
a laboratory the contents of the green ampoule could be
transferred to a cuvette and the absorbance determined in
a spectrophotometer at $A_{620nm}$. The results of an assay on
neonatal blood spots are shown in Table 3 reproduced herein
below. For the purposes of preliminary evaluation the colour
standard was set to give a $A_{620nm}$ value of 0.100.

TABLE 3

| Sample | Caputre Antibody | Trypsin Level[1] | Tag Antibody | EIA[2] |
|---|---|---|---|---|
| A | Monoclonal D | 55 ng/l | HRPO Monoclonal F | 0.05 |
| B[3] | Monoclonal D | 452 | HRPO Monoclonal F | 0.523 |
| C[3] | Monoclonal D | 817 | HRPO Monoclonal F | 0.217 |
| A | Monoclonal E | 55 | HRPO Monoclonal D | 0.05 |
| B[3] | Monoclonal E | 452 | HRPO Monoclonal D | 0.823 |
| C[3] | Monoclonal E | 817 | HRPO Monoclonal D | 0.410 |

1.        The level of trypsin was independently assessed by
the method of Crossley et al Clinica Chimica Acta 113. 111-121,
1981

2.        As previously stated, absorbance readings are not
required and the assay can be read visually by inexperienced
personnel.

3. Blood spots from patients later diagnosed as having the disease cystic fibrosis.

## EXAMPLE 4

The detection of human haemoglobin using MAb coated reaction probes.

In this example reaction probes were coated with human haemoglobin specific monoclonal antibodies able to capture human haemoglobin from a suitable solution and the captured antigen immobilised on the reaction probe was then subsequently tagged using a suitably labelled second anti-human haemoglobin monoclonal antibody.

Monoclonal antibodies to human haemoglobin were prepared in accordance with the technique established by Galfre et al, Nature 266, 550-2, 1977 as follows:

(i) Immunization of BALB/c mice with human haemoglobin Sgima Cat. No. H7379.

(ii) Fusing splenic cells from immunised mice obtained in Step (i) with NS-1 mouse myeloma cells where polyethylene glycol is used as a cell fusing agent to produce the hybridoma cells.

(iii) Propagating the hybridoma cells in tissue culture to produce anti-human haemoglobin monoclonal antibody secreting clones. Clones H, I and J did not crossreact with bovine, ovine, purcine lapine and avian haemoglobin.

(iv) Injecting the cloned hybridoma cells obtained in step (iii) into BALB/c mice wherein tumours are produced which secrete the anti-human haemoglobin monoclonal antibody.

(v) Collecting of monoclonal antibody from mice injected in step (iv).

Using this technique the following anti-human haemoglobin monoclonal antibodies were prepared:

      H   Clone C.49.25 1A2/99

      I         C.49.25 1A5/140

      J         C.49.25,3D4/60

      K         C.50.25,1D6/23

Reaction probes were coated with the above monoclonal antibodies as described previously. The assay kit was as described in example 1.

Procedure A

Before the test for human haemoglobin can be carried out some kit preparation is required.

1.       20 ml of distilled watter was added to fill the dropper bottle containing paper strip and buffer powder. The nozzle is inserted and the contents mixed.

2.       10 drops of buffer was added to the first empty tube. 50µl of the test sample was then transferred with the aid of the dilutor and the contents were mixed by agitation for 10 sec.

3.       20 drops of buffer were added to the red capped tube and contents mixed by inversion.

Assay Procedure

The test was carried out by the following required steps:

1.       The MAb coated probe was removed from its satchet and placed in the tube containing the diluted test sample. Leave 45 minutes.

2,3,4   Buffer was added to the neck of the second empty test tube and the probe was transferred to this tube for washing. After rotation for 30 seconds the probe was allowed to stand for 30 seconds. The probe was then removed and the contents of this tube discarded. The tube was again filled with fresh buffer. Thorough washing is essential. The washing procedure was repeated 2 more times.

5.       Transfer probe to red capped tube and leave 45 minutes.

6,7,8   Transfer probe to the third uncapped tube and proceed as for 2,3,4.

9. Transfer probe to the freen ampoule and leave 30 sec.

10. Transfer probe to yellow ampoule, rotate probe 30 sec and leave 5 minutes.

The probe was discarded after 10 minutes.

When this ampoule was held against a clean white background the presence of a blue colour greater then the colour standard (pink ampoule) was determined. If the test sample contained the specific antigen human haemoglobin a blue colour was obtained which ranged from a faint to a dark blue and the degree of colour was proportional to the amount of human haemoglobin present. If a quantitative result was required in a laboratory the contents of the green ampoule could be transferred to a cuvette and the absorbance determined in a spectrophotometer at $A_{620nm}$. The results of an assay on samples containing human haemoglobin are shown in Table 4 reproduced hereinbelow. For the purposes of preliminary evaluation the colour standard was set to give a $A_{620nm}$ value of 0.100.

TABLE 4

| Sample | Capture Antibody | Haemoglobin Level (1) | Tag Antibody | EIA |
|--------|------------------|-----------------------|--------------|-----|
| A | Monoclonal H | 0 | HRPO Monoclonal K | 0.05 |
| A | Monoclonal I | 0 | HRPO Monoclonal K | 0.04 |
| A | Monoclonal I | 0 | HRPO Monoclonal H | 0.06 |
| B | Monoclonal H | 500ng/ml | HRPO Monoclonal K | 0.54 |
| B | Monoclonal I | 500ng/ml | HRPO Monoclonal K | 0.32 |
| B | Monoclonal 1 | 500ng/ml | HRPO Monoclonal H | 0.86 |

(1) Haemoglobin level was determined by the addition of a known amount of human haemoglobin to the test sample.

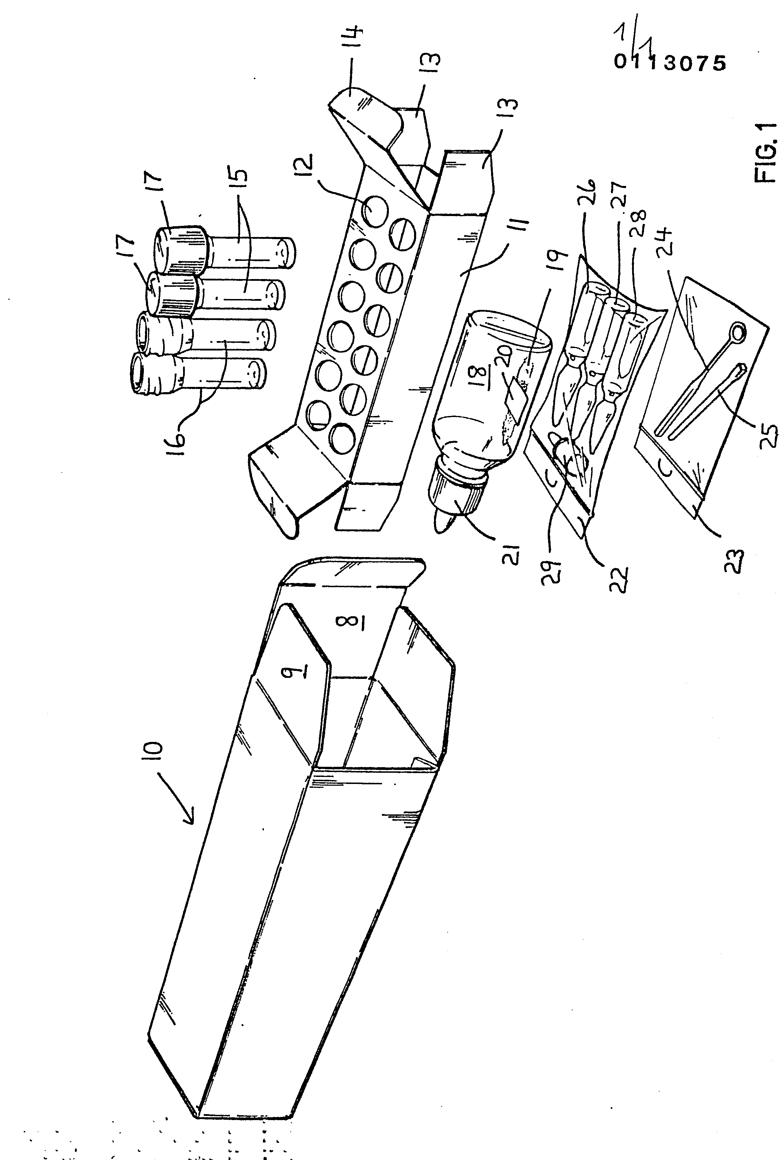

In FIG 1 of the attached drawing there is shown an outer container 10 for a reaction kit or system illustrative of the present invention. Container 10 has side flaps 9 and closure flap 8.

There is also shown inner container 11 having a

-28-

plurality of holes or apertures 12 in a top wall thereof. Inner container 11 also has at each end side flaps 13 and closure flap 14.

There is also shown a pair of tubes 15 having caps 17 which may have different colours for identification purposes. Also shown is a pair of test tubes 16 which are uncapped and which may be used for washing purposes.

Also shown is a bottle 18 containing buffer powder 19 and filter paper 20 inpregnated with detergent. Also shown is bag 23 formed from clear plastics material containing loop dilutor 24 and probe 25 having monoclonal antibody coated thereon. Also shown is plastics bag 22 containing ampoules 26,27,28 and dropper 29 which may be fitted within bottle 18 upon removal of cap 21 and thus the bottle 18 may be used as dropper. In this context the neck of bottle 18 may be internally threaded as well as externally threaded.

In operation the tubes 15 and 16 are supported in apertures 12. Bags 22 and 23 may then be opened. Ampoule 26 may contain hydrogen pentade. Ampoule 27 may contain substrate and ampoule 28 may contain a liquid indicating a suitable colour standard.

The second monoclonal antibody may be contained in one of the capped tubes 17 with the other capped tube being used as a sample container. The assay may then be carried out as described in Example 1.

It will be appreciated from the drawing that container 11 may fit neatly within outer container 10 upon closure of flaps 8 and 9. It also will be appreciated that bags 22 and 23 may fit within the interior of inner container 11 as well as bottle 18. The tubes 15 and 16 may also be retained within an accommodating aperture 12. Thus the whole assembly may be compactly packaged for storage or transportation.

Thus from the foregoing it will be appreciated that the field immunoassay method of the invention may provide substantial advantages in comparison to the prior art discussed above.

Thus by employing a coating restricted to monoclonal antibody compared to the polyclonal or multi site species used in the prior art two site assay systems referred to above,this allows the potention of the two site assay to be fully exploited. By employing hybridoma technology as established in Galfre et al Nature 266 550-2 1977 this has allowed the production of virtually unlimited quantities of 'capture' and 'tag' MAb of unparalleled antigen specificity and antigenic determinant specificity. The key provided by hybridoma technology resides in the production of MAb that can be employed specifically as the 'capture' MAb, which bind a unique antigenic determinant only present on the test Ag and absent on any other molecular species. Thus MAb 'capture-tag' assays can now be constructed in accordance with the present invention that were previously impossible due to the lack of Ag determinant specificity with conventionally produced polyclonal antisera (PAb), which resulted in cross-reactions with molecules having similar antigenic determinants to the test Ag.

Thus it is believed that the advent of the present invention provides a diagnostic method suitable for use under adverse conditions in the field which alleviates the above described disadvantages associated with the prior art (ie) the elongate member is simple to wash, can be used as a single assay, is economical in the use of biological reagents and does not require purified antigen. Also by employing MAb technology, inexhaustible supplies of Ag specific 'capture' and 'tag' MAb can be provided and electric or electronic signal amplification is not required to read the assay.

It is further believed that the diagnostic aid of the invention while being uniquely suited to employ the Two Site Immunoassay system using MAb technology is not necessarily restricted to using a second labelled 'tagging' MAb to generate a signal, but under certain instances the Ag 'captured' by the MAb coupled to the elongate member, may be itself capable of generating a suitable signal under the appropriate conditions.

The features disclosed in the foregoing description, in the following claims and/or in the accompanying drawings may, both separately and in any combination thereof, be material for realising  the invention in diverse forms thereof.

CLAIMS

1.      A field immunoassay reaction system for "in situ" testing including:

a dipstick probe or carrier at least partly coated with monoclonal antibody specific for an antigen contained in a body substance or fluid;

a plurality of containers;

detergent or other suitable substance to prevent non specific binding of antigen to the monoclonal antibody;

buffer and

"in situ" signal amplification or detection means.

2.      A reaction system as claimed in claim 1 further including a second monoclonal antibody labelled with an enzyme and an enzyme substrate.

3.      A reaction system as claimed in claim 2 wherein the enzyme is a peroxidase and hydrogen peroxide is also included as a co factor.

4.      A reaction system as claimed in any preceding claim wherein at least some of the plurality of containers have identifying means associated therewith to identify individual containers.

5.      A reaction system as claimed in claim 4 wherein there is provided one or more containers for carrying out washing steps in the field assay.

6.      A reaction system as claimed in any preceding claim wherein there is provided one or more loop dilutors.

7.      A reaction system as claimed in any preceding claim wherein there is also provided a colour standard or portable colourimeter.

8.      A reaction system as claimed in any preceding claim wherein the second monoclonal antibody is freezed dried and one of the containers contains diluent for said second monoclonal antibody to reconstitute same when required.

-31-

9.      A method of field immunoassay using the reaction system of claim 1 including the steps of:

(i)      transferring sample to be assayed into a container;

(ii)     inserting the dipstick probe into the container having the sample through an open top thereof;

(iii)    removing the probe from the sample container and subsequently washing the probe in one or more containers containing diluent wherein the probe is inserted into said one or more containers through an open top thereof and is subsequently removed;

(iv)     detecting the presence of an antigen in the sample by subjecting the probe to a signal amplification step.

10.     A method as claimed in claim 9 including the further steps of -

(v)      inserting the dipstick probe after step (iii) into a container containing enzyme labelled second monoclonal antibody;

(vi)     washing the dipstick probe in one or more containers containing diluent;  and

(vii)    inserting the dipstick probe into a container containing enzyme substrate before carrying out step (iv).

11.     A dipstick probe or carrier at least partly coated with monoclonal antibody (Mab) specific for an antigen contained in a body substance or fluid, suitable for use in the field immunoassay reaction system of any one of claims 1 to 8 and/or in the method of claim 9 or 10.

FIG. 1

1/1
0113075